# EUROPEAN PATENT SPECIFICATION

(11) **EP 3 729 096 B1**
(45) Date of publication and mention of the grant of the patent: **15.07.2026**
(21) Application number: 18829777.4
(22) Date of filing: 13.12.2018
(51) Int. Cl.: C07K 16/18, G01N 33/575, G01N 33/5752

(54) **SPARC ASSAY FOR DETECTING LUNG CANCER**
SPARC-TEST UM NACHWEIS VON LUNGENKREBS
TEST SPARC POUR LE DÉTECTION DU CANCER DU POUMON

(30) Priority: 19.12.2017 GB 201721308
(43) Date of publication of application: 28.10.2020
(73) Proprietor: Nordic Bioscience A/S, 2730 Herlev (DK)
(72) Inventor: KEHLET, Stephanie, Nina, 1812 Frederiksberg (DK); ØRSNES-LEEMING, Diana, 2730 Herlev (DK); KARSDAL, Morten, 2100 København Ø (DK)
(74) Representative: Beck Greener LLP
(86) International application number: PCT/EP2018/084841
(87) International publication number: WO 2019/121345

(56) References cited:
- WO-A1-2011/123395
- US-A1- 2011 319 405
- YAN HUANG ET AL: "SPARC expression and prognostic value in non-small cell lung cancer", AIZHENG - CHINESE JOURNAL OF CANCER, 10 October 2012 (2012-10-10), CN, XP055559768, ISSN: 1000-467X, DOI: 10.5732/cjc.012.10212
- ZHIPING ZHANG ET AL: "Correlation of KLF4 and SPARC expression with the clinical characteristics of non-small cell lung cancer", ZHONGGUO FEI AI ZA ZHI = CHINESE JOURNAL OF LUNG CANCER, 1 December 2012 (2012-12-01), China, pages 720 - 724, XP055559772, Retrieved from the Internet <URL:https://pdfs.semanticscholar.org/d8f5/0b2bda6f1a4ea01aca8fe5499bacfa1024a7.pdf?_ga=2.75776277.1884645914.1550679132-1474950065.1548749915> [retrieved on 20190220], DOI: 10.3779/j.issn.1009-3419.2012.12.05

## Description

### Field of the Invention

The present invention relates to an assay for detecting secreted proteome acidic and rich in cysteine (SPARC), and more specifically to its use in evaluating lung cancer.

### Background Art

Secreted proteome acidic and rich in cysteine (SPARC), also referred to as osteonectin or basement membrane protein 40 (BM-40), is a 32-kDa matricellular protein regulating extracellular matrix (ECM) assembly and deposition, growth factor signaling and interactions between cells and their surrounding ECM [1,2]. The expression of SPARC is elevated during embryonic development and is decreased in normal adult tissues. However, its expression is increased in epithelial/endothelial cells with a high ECM turnover, during abnormal tissue growth associated with neoplasia and during tissue injury and inflammation, highlighting the importance of SPARC in tissue remodeling [3-5].

SPARC has been shown to have chaperone like activity by inhibiting thermal aggregation of alcohol dehydrogenase in a concentration-dependent manner [6]. Furthermore, several studies have shown that SPARC binds different collagens in the ECM and is important for correct collagen deposition and assembly [7-13]. The chaperone activity of SPARC is regulated by different factors. A moderate extracellular concentration of Ca²⁺ has been shown to be necessary for binding of SPARC to its ECM partners. The presence of extracellular proteases is another important switch in the regulation of its collagen binding activity. Studies have shown that different metalloproteinases (MMP's) can cleave SPARC at a specific site which increases the affinity for collagens up to 20-fold [14, 15]. Interestingly, SPARC has been shown to increase the expression of MMP's in fibroblasts [16-18] causing a positive feedback loop. If this feedback mechanism becomes uncontrolled, it might be involved in the pathology of fibrotic disorders with increased collagen deposition.

Fibrosis is a part of the pathology and/or an end-point in many diseases such as cancer, hypertension, liver cirrhosis and fibrotic lung disorders. Fibrosis is characterized by an increased deposition of ECM, including collagens, which interferes with normal tissue function leading to organ failure. SPARC is known to be an important factor for fibrogenesis [19-23]. Wild-type mice with bleomycin induced pulmonary fibrosis have been shown to have an increased amount of collagens within the lungs compared to SPARC-null mice suggesting a higher fibrotic response occurring when SPARC is present [19, 20]. Furthermore, SPARC expression has been shown to be upregulated in fibrosis and cancer [24-27].

WO2011/123395 discloses a method of assessing whether a non-small cell lung cancer will respond to treatment comprising assessing differential levels of secreted protein, acididc and rich in cystine (SPARC) in tumour tissue. US2011/3194105 describes the upregulation of SPARC expression in IPF lung fibroblasts. The expression of SPACRC in NSCLS lung tissue was investigated by Huang et al [31]. Zhang et al [32] also teaches that SPARC in NSCLC lung tissue is increased as compared to control tissue and the expression is correlated with tumorigenesis and prognosis.

### Summary of the invention

The present inventors have now developed a highly sensitive SPARC assay that correlates, to a high degree, with patients suffering from lung cancer. The assay can distinguish between lung cancer and other fibrotic diseases and thus shows excellent diagnostic utility in the evaluation of lung cancer.

Accordingly, in a first aspect the present invention relates to a method of immunoassay for detecting and/or monitoring the progression of lung cancer in a patient, the method comprising contacting a patient biofluid sample with a monoclonal antibody specifically reactive with an N-terminus amino acid sequence LLARDFEKNY (SEQ ID NO: 1), wherein the patient biofluid sample is blood, urine, synovial fluid, serum or plasma, wherein the monoclonal antibody does not specifically recognise or bind an N-extended elongated version of said N-terminus amino acid sequence or an N-truncated shortened version of said N-terminus amino acid sequence, determining the amount of binding between said monoclonal antibody and peptides comprising said N-terminus amino acid sequence, and correlating said amount of binding with values associated with normal healthy subjects and/or values associated with known lung cancer severity and/or values obtained from said patient at a previous time point and/or a predetermined cut-off value.

As noted above, the monoclonal antibody does not specifically recognise or bind an N-extended elongated version of said N-terminus amino acid sequence or an N-truncated shortened version of said N-terminus amino acid sequence. In this regard "N-extended elongated version of said N-terminus amino acid sequence" means one or more amino acids extending beyond the N-terminus of the sequence H₂N-LLARDFEKNY (SEQ ID NO: 1). For example, if the N-terminal amino acid sequence H₂N-LLARDFEKNY (SEQ ID NO: 1) was elongated by a glutamic acid residue then the corresponding "N-extended elongated version" would be H₂N-ELLARDFEKNY (SEQ ID NO: 2). Similarly, "N-truncated shortened version of said N-terminus amino acid sequence" means one or more amino acids removed from the N-terminus of the sequence H₂N-LLARDFEKNY(SEQ ID NO: 1). For example, if the N-terminal amino acid sequence H₂N-LLARDFEKNY (SEQ ID NO: 1) was shortened by one amino acid residue then the corresponding "N-truncated shortened version" would be H₂N-LARDFEKNY (SEQ ID NO: 3).

The predetermined cut-off value is preferably at least 9.0 ng/mL, more preferably at least 15.0 ng/mL, even more preferably at least 20.0 ng/mL, even more preferably at least 25.0 ng/mL, and most preferably at least 30 ng/mL. In this regard, through the combined use of various statistical analyses it has been found that a measured amount of binding between the monoclonal antibody (described above) and the N-terminus biomarker of at least 9 ng/mL or greater may be determinative of the presence of lung cancer. By having a statistical cutoff value of at least 9 ng/mL, more preferably at least 15.0 ng/mL, even more preferably at least 20.0 ng/mL, even more preferably at least 25.0 ng/mL, and most preferably at least 30 ng/mL, it is possible to utilise the method of the invention to diagnose lung cancer with a high level of confidence. Or, in other words, applying the statistical cutoff value to the method of the invention is particularly advantageous as it results in a standalone diagnostic assay; i.e. it removes the need for any direct comparisons with healthy individuals and/or patients with known disease severity in order to arrive at a diagnostic conclusion. This may also be particularly advantageous when utilising the assay to evaluate patients that already have medical signs or symptoms that are generally indicative of lung cancer (e.g. as determined by a physical examination and/or consultation with a medical professional) as it may act as a quick and definitive tool for corroborating the initial prognosis and thus potentially remove the need for more invasive procedures, such as endoscopy or biopsy, and expedite the commencement of a suitable treatment regimen. In the particular case of lung cancer, an expedited conclusive diagnosis may result in the disease being detected at an earlier stage, which may in turn improve overall chances of survival.

The patient biofluid sample is selected from blood, urine, synovial fluid, serum or plasma.

The immunoassay may be, but is not limited to, a competition assay or a sandwich assay. Similarly, the immunoassay may be, but is not limited to, an enzyme-linked immunosorbent assay or a radioimmunoassay.

In a second aspect, the present invention relates to a method for determining whether a patient is responding positively to a treatment for lung cancer, wherein said method comprises using the method described supra to quantify the amount of peptides comprising the N-terminus amino acid sequence LLARDFEKNY (SEQ ID NO: 1) in at least two biological samples, said biological samples having been obtained from said patient at a first time point and at at least one subsequent time point during a period of administration of the treatment to said patient, and wherein a reduction in the quantity of peptides comprising the N-terminus amino acid sequence LLARDFEKNY (SEQ ID NO: 1) from said first time point to said at least one subsequent time point during the period of treatment is indicative of said patient responding positively to said treatment.

The above method may also be used to determine the efficacy of a novel therapeutic for treating lung cancer. In that regard, a novel therapeutic will be considered efficacious if the quantity of peptides comprising the N-terminus amino acid sequence LLARDFEKNY (SEQ ID NO: 1) is reduced from the said first time point to said at least one subsequent time point during the period of treatment using said novel therapeutic.

### Definitions

As used herein the term "N-terminus" refers to the extremity of a polypeptide, i.e. at the N-terminal end of the polypeptide, and is not to be construed as meaning in the general direction thereof.

As used herein the term "competitive ELISA" refers to a competitive enzyme-linked immunosorbent assay and is a technique known to the person skilled in the art.

As used herein the term "sandwich immunoassay" refers to the use of at least two antibodies for the detection of an antigen in a sample, and is a technique known to the person skilled in the art.

As used herein the term "amount of binding" refers to the quantification of binding between antibody and biomarker, which said quantification is determined by comparing the measured values of biomarker in the biofluid samples against a calibration curve, wherein the calibration curve is produced using standard samples of known concentration of the biomarker. In the specific assay disclosed herein which measures in biofluids the N-terminus biomarker having the N-terminus amino acid sequence LLARDFEKNY(SEQ ID NO: 1), the calibration curve is produced using standard samples of known concentration of the calibration peptide LLARDFEKNY (SEQ ID NO:1). The values measured in the biofluid samples are compared to the calibration curve to determine the actual quantity of biomarker in the sample. The present invention utilises spectrophotometric analysis to both produce the standard curve and measure the amount of binding in the biofluid samples; in the Examples set out below the method utilises HRP and TMB to produce a measurable colour intensity which is proportional to the amount of binding and which can be read by the spectrophotometer. Of course, any other suitable analytical method could also be used.

As used herein the "cut-off value" means an amount of binding that is determined statistically to be indicative of a high likelihood of a lung cancer or IPF in a patient, in that a measured value of biomarker in a patient sample that is at or above the statistical cutoff value corresponds to at least a 70% probability, preferably at least an 80% probability, preferably at least an 85% probability, more preferably at least a 90% probability, and most preferably at least a 95% probability of the presence or likelihood of a lung cancer or IPF.

As used herein the term "values associated with normal healthy subjects and/or values associated with known disease severity" means standardised quantities of SPARC determined by the method described supra for subjects considered to be healthy, i.e. without a lung cancer or IPF and/or standardised quantities of SPARC determined by the method described supra for subjects known to have a lung cancer or IPF of a known severity.

As used herein, "SPARC-M ELISA" refers to the specific competitive ELISA disclosed herein which quantifies in a sample the amount peptides having the N-terminus amino acid sequence LLARDFEKNY (SEQ ID NO:1).

### Figures

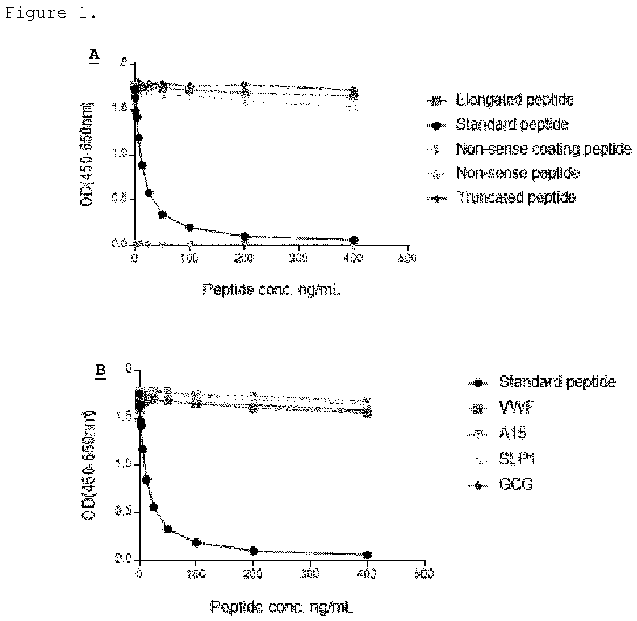

**Figure 1****. *Specificity of the SPARC-M monoclonal antibody:*** Monoclonal antibody reactivity towards (A) the standard peptide (LLARDFEKNY; SEQ ID NO: 1), the elongated peptide (ELLARDFEKNY; SEQ ID NO: 2), the truncated peptide (LARDFEKNY; SEQ ID NO: 3) a non-sense peptide (VPKDLPPDTT; SEQ ID NO: 4) and a non-sense coating peptide (VPKDLPPDTT-biotin; SEQ ID NO: 5); and (B) Von Willebrand factor (VWF), ADAMTS15 (A15), SPARC-like protein 1 (SLP1) and glucagon (GCG), were tested for in the competitive SPARC-M ELISA assay. Signals are shown as optical density (OD) at 450 nm (subtracted the background at 650 nm) as a function of peptide concentration.

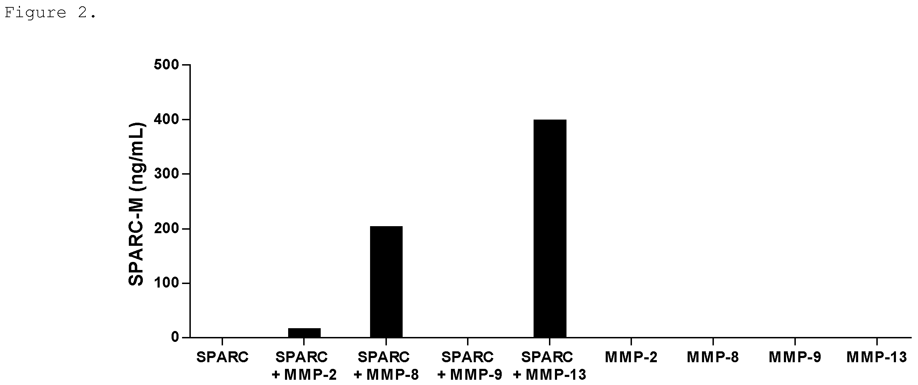

**Figure 2****. *Cleavage of SPARC by collagenases:*** SPARC was incubated with different MMP's and SPARC-M levels were measured after 24 hours. Data were normalized by subtracting the background measured in buffer alone. The graph below is representative of two experiments.

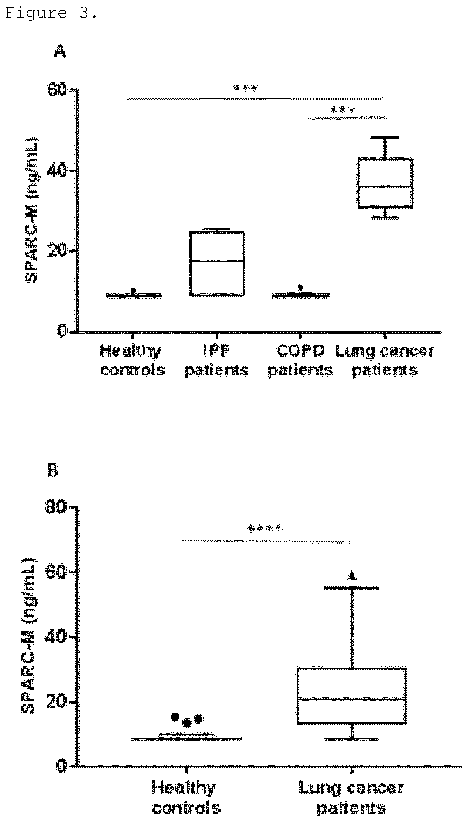

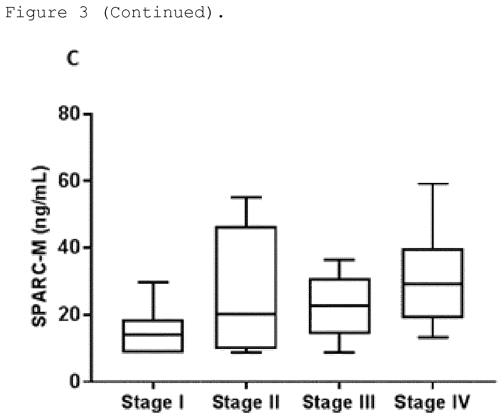

**Figure 3****. *Serum SPARC-M levels* in *patients with fibrotic disorders and healthy controls:*** (A) Cohort 1: Serum SPARC-M was assessed in healthy controls (n=6), IPF patients (n=7), COPD patients (n=8) and lung cancer patients (n=8). Groups were compared using Kruskal-Wallis adjusted for Dunn's multiple comparisons test; (B) Cohort 2: Serum SPARC-M was assessed in healthy controls (n=20) and lung cancer patients (n=40). Groups were compared using unpaired, two-tailed Mann-Whitney test; (C) Lung cancer patients (from cohort 2) were stratified according to their cancer stage (stage I-IV, n=10 in each group). Data were compared using one-way ANOVA adjusted for Tukey's multiple comparisons test. All Data are shown as Tukey box plots. Significance level: ***: p<0.001, ****: p<0.0001.

### Examples

The presently disclosed embodiments are described in the following Examples, which are set forth to aid in the understanding of the disclosure, and should not be construed to limit in any way the scope of the disclosure as defined in the claims which follow thereafter. The following examples are put forth so as to provide those of ordinary skill in the art with a complete disclosure and description of how to make and use the described embodiments, and are not intended to limit the scope of the present disclosure nor are they intended to represent that the experiments below are all or the only experiments performed. Efforts have been made to ensure accuracy with respect to numbers used (e.g. amounts, temperature, etc.) but some experimental errors and deviations should be accounted for. Unless indicated otherwise, parts are parts by weight, molecular weight is weight average molecular weight, temperature is in degrees Centigrade, and pressure is at or near atmospheric.

In the following examples, the following materials and methods were employed.

### Development of SPARC-M ELISA

### Selection of peptides

The following cleavage site (↓) on SPARC was previously identified by Edman degradation [14]:
₂₁₁HPVE ↓ LLARDFEKNYNMYIFP₂₃₀. To generate an antibody specific for the N-terminal of the cleavage fragment, a sequence of 10 amino acids adjacent to the site was chosen as the target: ↓₂₁₅LLARDFEKNY₂₂₄ (SEQ ID NO: 1). The sequence was blasted for homology to other human secreted extracellular matrix proteins using NPS@: Network Protein Sequence Analysis with the UniprotKB/Swiss-prot database [29].

Synthetic peptides used for monoclonal antibody production and validation of the ELISA assay were purchased from Genscript (Piscataway, NJ, USA) and shown in Table 1.

**Table 1. Synthetic peptides used for development and validation of the SPARC-M ELISA assay**

| **Peptide name** | **Amino acid sequence** | **SEQ ID NO** |
|---|---|---|
| Standard peptide | LLARDFEKNY | 1 |
| Immunogenic peptide | LLARDFEKNY-GGC-KLH | 6 |
| Biotinylated coating peptide | LLARDFEKNY-K-biotin | 7 |
| Elongated peptide | ELLARDFEKNY | 2 |
| Truncated peptide | LARDFEKNY | 3 |
| Non-sense standard peptide | VPKDLPPDTT | 4 |
| Non-sense coating peptide | VPKDLPPDTT-biotin | 5 |
| Von Willebrand factor (VWF) | LLARDCQDHS | 8 |
| Glucagon (GCG) | LAARDFINWL | 9 |
| SPARC-like protein 1 (SLP1) | LLLRDFKKNY | 10 |
| ADAMTS15 (A15) | LLARDQCNLH | 11 |

The target sequence was used as the standard peptide (LLARDFEKNY; SEQ ID NO: 1). A biotinylated peptide (LLARDFEKNY-K-biotin; SEQ ID NO:7) was included as a coating peptide with addition of a lysine residue to the C-terminal end to ensure biotin linking. The specificity of the antibody was tested by including an elongated standard peptide with an additional amino acid added to the N-terminal of the target peptide sequence (ELLARDFEKNY; SEQ ID NO: 1), a truncated standard peptide with a removal of the first N-terminal amino acid (LARDFEKNY; SEQ ID NO:3) as well as a non-sense standard peptide (VPKDLPPDTT; SEQ ID NO:4) and a non-sense biotinylated coating peptide (VPKDLPPDTT-biotin; SEQ ID NO:5) in the assay validation. Four peptides (Von Willebrand factor, glucagon, SPARC-like protein 1 and ADAMTS15) with one amino acid mismatch compared to the first six amino acids in the target sequence were also included to further test the antibody specificity. The immunogenic peptide (LLARDFEKNY-GGC-KLH; SEQ ID NO:6) was generated by covalently crosslinking the standard peptide to Keyhole Limpet Hemocyanin (KLH) carrier protein using Succinimidyl 4-(N-maleimidomethyl)cyclohexane-1-carboxylate, SMCC (Thermo Scientific, Waltham, MA, USA, cat.no. 22336). Glycine and cysteine residues was added at the C-terminal end to ensure right linking of the carrier protein.

### Monoclonal antibody production

Four to six week old Balb/C mice were immunized by subcutaneous injection of 200 µL emulsified antigen containing 50 µg immunogenic peptide (LLARDFEKNY-GGC-KLH; SEQ ID NO:6) mixed with Freund's incomplete adjuvant (Sigma-Aldrich, St. Louis, MO, USA). Consecutive immunizations were performed at 2-week intervals until stable sera titer levels were reached. The mouse with the highest titer rested for four weeks and was then boosted with 50 µg immunogenic peptide in 100 µL 0.9% NaCl solution intravenously. Hybridoma cells were produced by fusing spleen cells with SP2/0 myeloma cells as previously described [30]. The resultant hybridoma cells were then cultured in 96-well microtiter plates and standard limited dilution was used to secure monoclonal growth. The supernatants were screened for reactivity using the biotinylated peptide (LLARDFEKNY-K-biotin; SEQ ID NO:7) as coating agent in the competitive immunoassays.

### Clone characterization

The reactivity of the monoclonal antibody was evaluated by displacement using human serum samples and the standard peptide (LLARDFEKNY; SEQ ID NO:1) in a preliminary ELISA using 10 ng/mL biotinylated coating peptide on streptavidin-coated microtiter plates (Roche, Basel, Switzerland, cat. #11940279) and the supernatant from the antibody producing monoclonal hybridoma cells. The clone with the best reactivity towards the standard peptide was purified using protein-G-columns according to the manufacturer's instructions (GE Healthcare Life Sciences, Little Chalfont, UK, cat. #17-0404-01).

### SPARC-M ELISA protocol

Optimal incubation -buffer, -time and -temperature, as well as the optimal concentrations of antibody and coating peptide were determined and the finalized SPARC-M competitive ELISA protocol was as follows:
A 96-well streptavidin-coated microtiter plate was coated with 1.1 ng/mL biotinylated coating peptide dissolved in assay buffer (50 mM Tris-BTB, 4 g/L NaCl, pH 8.0) and incubated for 30 min. at 20°C in darkness shaking (300 rpm). Twenty µL standard peptide or pre-diluted serum (1:4) were added to appropriate wells, followed by the addition of 100 µL monoclonal antibody dissolved in assay buffer to a concentration of 14 ng/mL to each well and incubated 1 hour at 20°C in darkness shaking (300 rpm). One hundred µL of goat anti-mouse POD-conjugated IgG antibody (Thermo Scientific, Waltham, MA, USA, cat. #31437) diluted 1:6000 in assay buffer was added to each well and incubated 1 hour at 20°C in darkness shaking. All incubation steps were followed by five washes in washing buffer (20 mM Tris, 50 mM NaCl, pH 7.2). Finally, 100 µL tetramethylbenzidine (TMB) (cat. 438OH, Kem-En-Tec Diagnostics, Denmark) was added to each well and the plate was incubated for 15 minutes at 20°C in darkness shaking. The enzymatic reaction was stopped by adding 0.18 M H₂SO₄ and absorbance was measured at 450 nm with 650 nm as reference. A calibration curve was plotted using a 4-parameter logistic curve fit. Data were analyzed using the SoftMax Pro v.6.3 software.

### Technical evaluation of the SPARC-M ELISA

To evaluate the technical performance of the SPARC-M ELISA, the following validation tests were carried out: Inter- and intra-assay variation, linearity, lower limit of detection, upper limit of detection, analyte stability (freeze/thaw and storage) and interference.

The inter- and intra-assay variation was determined by ten independent runs on different days using seven quality control samples covering the detection range, with each run consisting of double-determinations of the samples. The seven quality control samples consisted of: two human serum samples and five samples with standard peptide in buffer. Intra-assay variation was calculated as the mean coefficient of variance (CV%) within plates and the inter-assay variation was calculated as the mean CV% between the ten individual runs analyzed on different days. To assess linearity of the assay, two-fold dilutions of human serum samples were performed and dilution linearity was calculated as a percentage of recovery of the un-diluted sample. The lower limit of detection (LLOD) was determined from 21 measurements using assay buffer as sample and was calculated as the mean + three standard deviations. The upper limit of detection (ULOD) was determined from ten independent runs of the highest standard peptide concentration and was calculated as the mean back-calibration calculation + three standard deviations. Analyte stability was first determined by the effect of repeated freeze/thaw of serum samples by measuring the SPARC-M level in three human serum samples in four freeze/thaw cycles. The freeze/thaw recovery was calculated with the first cycle as reference. Second, analyte stability in relation to storage was determined by a 48 hour study performed at 4°C or 20°C. The SPARC-M level in three human serum samples was measured after 0 h, 4h, 24h and 48 h of storage and recovery was calculated with samples stored at -20°C as reference. Interference was determined by adding a low/high content of hemoglobin (0.155/0.310 mM), lipemia/lipids (4.83/10.98 mM) and biotin (30/90 ng/mL) to a serum sample of known concentration. Recovery percentage was calculated with the serum sample as reference.

### Cleavage of SPARC in vitro

Recombinant human SPARC (PeproTech, New Jersey, USA, cat. #120-36) was reconstituted to a final concentration of 1000 ug/mL in MMP-buffer (50 mM Tris-HCl, 150 mM NaCl, 10 mM CaCl2, 10uM ZnCl, 0.05%Brij35, pH 7.5). MMP-2, MMP-8, MMP-9 and MMP-13 (Giotto, Firenze, Italy, cat. #G04MP02C, #G04MP08C, #G04MP09C, #G04MP13C) were added 1:10 (1 µg MMP and 10 µg SPARC). A positive control protein with known cleavage by the above proteases was included. The solutions incubated at 37°C for 24h. The reaction was stopped by adding 1 µM EDTA to the solutions. MMP-buffer with the different proteases were included as controls. Samples were stored at - 80°C until analysis. The activity of the proteases was confirmed by silverstaining according to the manufacturer's instructions (SilverXpress^{®}, Invitrogen, cat. #LC6100) and coomassie blue (data not shown).

### Clinical validation of SPARC-M

Patient serum samples were obtained from the commercial vendor ProteoGenex (Culver City, CA, USA). Cohort 1 consisted of patients with lung cancer, idiopathic pulmonary fibrosis (IPF), chronic obstructive pulmonary disease (COPD) and colonoscopy-negative controls with no symptomatic or chronic disease (Table 2). Cohort 2 included 40 men and women with different stages of lung cancer, and 20 age-matched colonoscopy-negative controls with no symptomatic or chronic disease (Table 2). Appropriate Institutional Review Board/Independent Ethical Committee approved sample collection and all subjects filed informed consent.

**Table 2. Clinical sample overview and patients demographics**

| **Cohort** | **Samples** | **No. of subjects** | **Mean age (range)** | **Gender, % females** | **Mean BMI (range)** | **Tumor stage I** | **Tumor stage II** | **Tumor stage III** | **Tumor stage IV** |
|---|---|---|---|---|---|---|---|---|---|
| 1 | Lung cancer patients | 8 | 61 (47-77) | 13 | - | - | - | - | - |
| 1 | IPF patients | 7 | 73 (55-81) | 57 | - | - | - | - | - |
| 1 | COPD patients | 8 | 75 (69-82) | 50 | - | - | - | - | - |
| 1 | Healthy controls | 6 | 55 (44-65) | 83 | - | - | - | - | - |
| 2 | Lung cancer patients | 40 | 62 (55-66) | 50 | 25 (16-35) | 10 | 10 | 10 | 10 |
| 2 | Healthy controls | 20 | 62 (60-65) | 50 | 26 (22-32) | - | - | - | - |

### Statistical analysis

The level of SPARC-M in serum samples was compared using unpaired, two-tailed Mann-Whitney test and Kruskal-Wallis adjusted for Dunn's multiple comparisons test. Patients were stratified according to their tumor stage and the level of SPARC-M in each group was compared using one-way ANOVA adjusted for Tukey's multiple comparisons test. D'Agostino-Pearson omnibus test was used to assess the normality of the data. The diagnostic power was investigated by the area under the receiver operating characteristics (AUROC).

Graph design and statistical analyses were performed using GraphPad Prism version 7 (GraphPad Software, Inc., CA, USA).

### Results

### Specificity of the SPARC-M ELISA assay

The target sequence, LLARDFEKNY (SEQ ID NO:1), was blasted for homology to other human secreted extracellular matrix proteins using NPS@: Network Protein Sequence Analysis with the UniprotKB/Swiss-prot database. The target sequence was found to be unique to human SPARC when compared to other secreted ECM proteins. Allowing one amino acid mismatch, four secreted extracellular matrix proteins, Von Willebrand factor, glucagon, SPARC-like protein 1 and ADAMTS15, were identified with mismatches at the 6^{th}, 2^{nd}, 3^{rd} and 6^{th} position, respectively (Table 1). There was no reactivity against the sequence of the four peptides (Figure 1B) suggesting high specificity of the antibody for the target sequence. The specificity of the competitive SPARC-M ELISA was further evaluated by analyzing the reactivity towards the standard peptide, a non-sense peptide, an elongated peptide, a truncated peptide and using a non-sense biotinylated coating peptide. All peptide sequences are shown in Table 1 and results are shown in Figure 1A. The antibody only reacted with the standard peptide and the standard peptide clearly inhibited the signal in a dose-dependent manner compared to the other peptides. No detectable signal was observed when using the non-sense biotinylated coating peptide.

Together, these data suggest that the selected antibody exhibits high neo-epitope specificity.

### Technical evaluation of the SPARC-M ELISA assay

The technical performance of the SPARC-M ELISA assay was further evaluated according to inter- and intra-assay variation, linearity, lower limit of detection, upper limit of detection, analyte stability (freeze/thaw and storage) and interference. The different validation steps and SPARC-M performance are shown in Table 3.

**Table 3. Technical validation data of the SPARC-M ELISA assay**

| | |
|---|---|
| **Technical validation step** | **SPARC-M performance** |
| Detection range (LLOD-ULOD) | 2.7 - 300.7 ng/mL |
| Intra-assay variation | 6% |
| Inter-assay variation | 10% |
| Dilution of serum samples | 1:4 |
| Dilution recovery (1:4 pre-dilution) | 96% (77-102%) |
| Freeze/thaw recovery (4 cycles) | 92% (86-103%) |
| Analyte stability up to 48h, 4°C and 4h, 20°C | 88% (84-96%) |
| Interference Lipids, low/high | 96%/97% |
| Interference Biotin, low/high | 96%/98% |
| Interference Hemoglobin, low/high | 96%/80% |

| | |
|---|---|
| Percentages are reported as mean with range shown in brackets | |

The measuring range (LLOD to ULOD) of the assay was determined to be 2.7 - 300.7 ng/mL. The intra- and inter-assay variation was 6% and 10%, respectively. The acceptance criterion was below 10% for the intra-assay variation and below 15% for the inter-assay variation and therefore acceptable. To obtain linearity, human serum needed to be diluted 1:4 and the mean dilution recovery for 1:4 pre-diluted human serum was 96%. The analyte stability was analyzed according to freeze/thaw cycles and storage stability at 4°C and 20°C with an acceptance criterion of the recovery within 100%±20%. The analyte recovery in serum was 92% after 4 freeze/thaw cycles. After storage at 4°C for 48 hours the recovery was 84%. Analyte stability was also tested at 20°C for 4, 24 and 48 hours. The recovery after 4 hours was 88%. However after 24 hours the analyte could not be recovered within the acceptance range (50% recovery). These data indicate that the analyte in serum is stable at 4°C up to 48 hours, however upon analysis serum samples should not be stored above 20°C for more than four hours. No interference was detected from either low or high contents of biotin, lipids or hemoglobin with recoveries ranging from 80-98%. The acceptance criterion was a recovery within 100%±20%.

### Degradation of SPARC by collagenases (MMP-8 and MMP-13)

To further evaluate the specificity of the antibody and to investigate which proteases generate SPARC-M, different gelatinases (MMP-2 and MMP-9) and collagenases (MMP-8 and MMP-13) were incubated with recombinant full-length SPARC. As shown in Figure 2, the collagenases were able to generate the fragment, with MMP-13 giving the highest level of SPARC-M. In contrast, no SPARC-M was generated without the proteases or when incubated with MMP-9. MMP-2 was able to generate a small amount of SPARC-M as compared to the collagenases.

These results indicate that the antibody is specific for the cleavage site and that collagenases compared to gelatinases have a higher preference for SPARC at this specific site.

### Clinical evaluation of SPARC-M

To investigate whether SPARC-M had clinical disease relevance and biomarker potential, SPARC-M was measured in patients with different fibrotic lung disorders and healthy controls. Cohort 1 consisted of patients with lung cancer, IPF, COPD and healthy controls. As shown in figure 3A, SPARC-M was significantly elevated in lung cancer patients compared to healthy controls and COPD patients. IPF patients also had an increased level of SPARC-M compared to healthy controls. To confirm these findings, SPARC-M was measured in a second and larger cohort including 40 lung cancer patients and 20 healthy controls. A significant increase in SPARC-M in lung cancer patients as compared to healthy controls was confirmed (Figure 3B). The area under the receiver operating characteristics (AUROC) was used to evaluate the discriminative power of SPARC-M in relation to lung cancer patients and healthy controls (cohort 2). SPARC-M was able to discriminate between patients and healthy controls with an AUROC of 0.87 (95%CI: 0.78-0.96).

To examine if the level of SPARC-M was different in patients with metastasis (high tumor burden) compared to patients with localized tumors, patients from cohort 2 were stratified according to their tumor stage (stage I-IV). No significant difference was observed between the tumor stages, however an increasing trend of SPARC-M was observed with increasing tumor stage (Figure 3C).

### Conclusion

The present study describes the development and biological validation of a competitive ELISA assay quantifying a fragment of SPARC in serum. The main findings of this study were: 1) the investigated fragment was detectable in serum and significantly elevated in lung cancer patients compared to healthy controls, 2) the assay was technically robust and specific towards a unique MMP-8/MMP-13 degraded fragment of SPARC, SPARC-M, and 3) the assay shows potential for use in evaluating IPF.

In this specification, unless expressly otherwise indicated, the word 'or' is used in the sense of an operator that returns a true value when either or both of the stated conditions is met, as opposed to the operator 'exclusive or' which requires that only one of the conditions is met. The word 'comprising' is used in the sense of 'including' rather than in to mean 'consisting of'.

### References

1. Lane TF, Sage EH. The biology of SPARC, a protein that modulates cell-matrix interactions. FASEB J. 1994;8:163-73.
2. Bradshaw AD. The role of SPARC in extracellular matrix assembly. Journal of Cell Communication and Signaling. 2009;3:239-46.
3. Brekken RA, Sage EH. SPARC, a matricellular protein: at the crossroads of cell-matrix. Matrix Biology. 2000;19:569-80.
4. Chiodoni C, Colombo MP, Sangaletti S. Matricellular proteins: From homeostasis to inflammation, cancer, and metastasis. Cancer and Metastasis Reviews. 2010;29:295-307.
5. Yan Q, Sage EH. SPARC, a Matricellular Glycoprotein with Important Biological Functions. J Histochem Cytochem. 1999;47:1495-505. doi:10.1177/002215549904701201.
6. Emerson RO, Sage EH, Ghosh JG, Clark JI. Chaperone-like activity revealed in the matricellular protein SPARC. J Cell Biochem. 2006;98:701-5.
7. Rosset EM, Bradshaw AD. SPARC/osteonectin in mineralized tissue. Matrix Biology. 2016;52-54:78-87.
8. Bradshaw AD, Baicu CF, Rentz TJ, Van Laer AO, Boggs J, Lacy JM, et al. Pressure overload-induced alterations in fibrillar collagen content and myocardial diastolic function: Role of secreted protein acidic and rich in cysteine (SPARC) in post-synthetic procollagen processing. Circulation. 2009;119:269-80.
9. Bradshaw AD, Puolakkainen P, Dasgupta J, Davidson JM, Wight TN, Sage EH. SPARC-null mice display abnormalities in the dermis characterized by decreased collagen fibril diameter and reduced tensile strength. J Invest Dermatol. 2003;120:949-55.
10. Delany AM, Amling M, Priemel M, Howe C, Baron R, Canalis E. Osteopenia and decreased bone formation in osteonectin-deficient mice. J Clin Invest. 2000;105:915-23.
11. Rentz TJ, Poobalarahi F, Bornstein P, Sage EH, Bradshaw AD. SPARC regulates processing of procollagen I and collagen fibrillogenesis in dermal fibroblasts. J Biol Chem. 2007;282:22062-71.
12. Trombetta-eSilva J. The Function of SPARC as a Mediator of Fibrosis. Open Rheumatol J. 2012;6:146-55.
13. Trombetta JM, Bradshaw AD, Johnson RH. SPARC/Osteonectin Functions to Maintain Homeostasis of the Collagenous Extracellular Matrix in the Periodontal Ligament. J Histochem Cytochem. 2010;58:871-9. doi:10.1369/jhc.2010.956144.
14. Sasaki T, Göhring W, Mann K, Maurer P, Hohenester E, Knäuper V, et al. Limited cleavage of extracellular matrix protein BM-40 by matrix metalloproteinases increases its affinity for collagens. J Biol Chem. 1997;272:9237-43.
15. Sasaki T, Miosge N, Timpl R. Immunochemical and tissue analysis of protease generated neoepitopes of BM-40 (osteonectin, SPARC) which are correlated to a higher affinity binding to collagens. Matrix Biol. 1999;18:499-508.
16. Gilles C, Bassuk J a, Pulyaeva H, Sage EH, Foidart JM, Thompson EW. SPARC/osteonectin induces matrix metalloproteinase 2 activation in human breast cancer cell lines. Cancer Res. 1998;58:5529-36.
17. Jacob K, Webber M, Benayahu D, Kleinman HK. Osteonectin promotes prostate cancer cell migration and invasion: A possible mechanism for metastasis to bone. Cancer Res. 1999;59:4453-7.
18. Tremble PM, Lane TF, Sage EH, Werb Z. SPARC, a secreted protein associated with morphogenesis and tissue remodeling, induces expression of metalloproteinases in fibroblasts through a novel extracellular matrix-dependent pathway. J Cell Biol. 1993;121:1433-44.
19. Sangaletti S, Tripodo C, Cappetti B, Casalini P, Chiodoni C, Piconese S, et al. SPARC oppositely regulates inflammation and fibrosis in bleomycin-induced lung damage. Am J Pathol. 2011;179:3000-10.
20. Strandjord TP, Madtes DK, Weiss DJ, Sage EH. Collagen accumulation is decreased in SPARC-null mice with bleomycin-induced pulmonary fibrosis. Am J Physiol. 1999;277 3 Pt 1:L628-35.
   http://www.ncbi.nlm.nih.gov/pubmed/10484471.
21. Wang J-C, Lai S, Guo X, Zhang X, de Crombrugghe B, Sonnylal S, et al. Attenuation of fibrosis in vitro and in vivo with SPARC siRNA. Arthritis Res Ther. 2010;12:1-9.
22. Pichler RH, Hugo C, Shankland SJ, Reed MJ, Bassuk JA, Andoh TF, et al. SPARC is expressed in renal interstitial fibrosis and in renal vascular injury. Kidney Int. 1996;50:1978-89.
23. Camino AM, Atorrasagasti C, Maccio D, Prada F, Salvatierra E, Rizzo M, et al. Adenovirus-mediated inhibition of SPARC attenuates liver fibrosis in rats. J Gene Med. 2008;10:993-1004.
24. Neuzillet C, Tijeras-Raballand A, Cros J, Faivre S, Hammel P, Raymond E. Stromal expression of SPARC in pancreatic adenocarcinoma. Cancer and Metastasis Reviews. 2013;32:585-602.
25. Wong SLI, Sukkar MB. The SPARC protein: an overview of its role in lung cancer and pulmonary fibrosis and its potential role in chronic airways disease. Br J Pharmacol. 2017;174:3-14.
26. Frizell E, Liu SL, Abraham A, Ozaki I, Eghbali M, Helene Sage E, et al. Expression of SPARC in normal and fibrotic livers. Hepatology. 1995;21:847-54.
27. Kuhn C, Mason RJ. Immunolocalization of SPARC, tenascin, and thrombospondin in pulmonary fibrosis. Am J Pathol. 1995;147:1759-69.
28. Leeming D, He Y, Veidal S, Nguyen Q, Larsen D, Koizumi M, et al. A novel marker for assessment of liver matrix remodeling: An enzyme-linked immunosorbent assay (ELISA) detecting a MMP generated type I collagen neo-epitope (C1M). Biomarkers. 2011;16:616-28. doi:10.3109/1354750X.2011.620628.
29. Combet C, Blanchet C, Geourjon C, Deléage G. NPS@: network protein sequence analysis. Trends Biochem Sci. 2000;25:147-50. http://www.ncbi.nlm.nih.gov/pubmed/10694887. Accessed 27 May 2016.
30. Gefter ML, Margulies DH, Scharff MD. A simple method for polyethylene glycol-promoted hybridization of mouse myeloma cells. Somatic Cell Genet. 1977;3:231-6.
31. Yan Huang, Jing Zhang, Yuan-Yuan Zhao, Wei Jiang, Cong Xue, Fei Xu, Hong-Yun Zhao, Yang Zhang, Li-Ping Zhao, Zhi-Huang Hu, Zhi-Wen Yao, Qian-Yong Liu, Li Zhang. SPARC expression and prognostic value in non-small cell lung cancer Chin J Cancer (2012) Nov;31(11):541-8
32. Zhang Z, Wang Z , Liu X , Shi M , Chen G , Zhang B , Li Z , Song L. Correlation of KLF4 and SPARC expression with the clinical characteristics of non-small cell lung Chinese Journal of Lung Cancer (2012) 15(12):720-724.

## Claims

1. A method of immunoassay for detecting and/or monitoring progression of lung cancer in a patient, the method comprising contacting a patient biofluid sample with a monoclonal antibody specifically reactive with an N-terminus amino acid sequence LLARDFEKNY (SEQ ID NO:1), wherein the patient biofluid sample is blood, urine, synovial fluid, serum or plasma, wherein the monoclonal antibody does not specifically recognise or bind an N-extended elongated version of said N-terminus amino acid sequence or an N-truncated shortened version of said N-terminus amino acid sequence, determining the amount of binding between said monoclonal antibody and peptides comprising said N-terminus amino acid sequence, and correlating said amount of binding with values associated with normal healthy subjects and/or values associated with known lung cancer severity and/or values obtained from said patient at a previous time point and/or a predetermined cut-off value.

2. The method as claimed in claim 1, wherein the predetermined cut-off value is at least 9.0 ng/mL.

3. The method of claims 1 or 2, wherein the immunoassay is a competition assay or a sandwich assay.

4. The method of claims 1 to 3, wherein the immunoassay is an enzyme-linked immunosorbent assay or a radioimmunoassay.

5. A method for determining whether a patient is responding positively to a treatment for lung cancer, wherein said method comprises using the method of claims 1 to 4 to quantify the amount of peptides comprising the N-terminus amino acid sequence LLARDFEKNY (SEQ ID NO: 1) in at least two biological samples, said biological samples having been obtained from said patient at a first time point and at at least one subsequent time point during a period of administration of the treatment to said patient, and wherein a reduction in the quantity of peptides comprising the N-terminus amino acid sequence LLARDFEKNY (SEQ ID NO: 1) from said first time point to said at least one subsequent time point during the period of treatment is indicative of said patient responding positively to said treatment.

## Patentansprüche

1. Immunoassayverfahren zum Nachweisen und/oder Überwachen der Progression von Lungenkrebs in einem Patienten, wobei das Verfahren ein Inkontaktbringen einer Patientenprobe eines biologischen Fluids mit einem monoklonalen Antikörper, der spezifisch reaktiv mit einer N-Terminus-Aminosäuresequenz LLARDFEKNY (SEQ ID Nr. 1) ist, wobei die Patientenprobe des biologischen Fluids Blut, Urin, Gelenkflüssigkeit, Serum oder Plasma ist, wobei der monoklonale Antikörper eine N-erweiterte verlängerte Version der besagten N-Terminus-Aminosäuresequenz oder eine N-gestutzte verkürzte Version der besagten N-Terminus-Aminosäuresequenz nicht spezifisch erkennt oder bindet, ein Bestimmen des Umfangs der Bindung zwischen dem besagten monoklonalen Antikörper und Peptiden, die die besagte N-Terminus-Aminosäuresequenz umfassen, und ein Korrelieren des besagten Umfangs der Bindung mit Werten, die mit normalen gesunden Probanden assoziiert sind, und/oder Werten, die mit einem bekannten Lungenkrebsschweregrad assoziiert sind, und/oder Werte, die von dem besagten Patienten zu einem vorherigen Zeitpunkt erhalten wurden, und/oder einem vorbestimmten Cut-Off-Wert umfasst.

2. Verfahren nach Anspruch 1, wobei der vorbestimmte Cut-Off-Wert mindestens 9,0 ng/mL beträgt.

3. Verfahren nach den Ansprüchen 1 oder 2, wobei der Immunoassay ein Kompetitionsassay oder ein Sandwich-Assay ist.

4. Verfahren nach den Ansprüchen 1 bis 3, wobei der Immunoassay ein Enzyme-Linked Immunosorbent Assay oder ein Radioimmunoassay ist.

5. Verfahren zum Bestimmen, ob ein Patient positiv auf eine Behandlung von Lungenkrebs anspricht, wobei das besagte Verfahren ein Verwenden des Verfahrens nach den Ansprüchen 1 bis 4 umfasst, um die Menge von Peptiden, die die N-Terminus-Aminosäuresequenz LLARDFEKNY (SEQ ID Nr. 1) umfassen, in mindestens zwei biologischen Proben zu quantifizieren, wobei die besagten biologischen Proben von dem besagten Patienten zu einem ersten Zeitpunkt und zu mindestens einem anschließenden Zeitpunkt während einer Verabreichungsperiode bezogen wurden, und wobei eine Verringerung der Quantität von Peptiden, die die N-Terminus-Aminosäuresequenz LLARDFEKNY (SEQ ID Nr. 1) umfassen, von dem besagten ersten Zeitpunkt zu dem besagten mindestens einen anschließenden Zeitpunkt während der Behandlungsperiode darauf hinweist, dass der besagte Patient positiv auf die besagte Behandlung anspricht.

## Revendications

1. Procédé d'immunodosage pour détecter et/ou surveiller la progression d'un cancer du poumon chez un patient, le procédé comprenant la mise en contact d'un échantillon de biofluide provenant d'un patient avec un anticorps monoclonal spécifiquement réactif vis-à-vis d'une séquence d'acides aminés N-terminale LLARDFEKNY (SEQ ID NO:1), dans lequel l'échantillon de biofluide du patient est du sang, de l'urine, du liquide synovial, du sérum ou du plasma, dans lequel l'anticorps monoclonal ne reconnaît pas spécifiquement et ne se lie à une version allongée en N-terminal de ladite séquence d'acides aminés N-terminale ni à une version tronquée en N-terminal de ladite séquence d'acides aminés N-terminale, la détermination de la quantité de liaison entre ledit anticorps monoclonal et des peptides comprenant ladite séquence d'acides aminés N-terminale, et la corrélation de ladite quantité de liaison avec des valeurs associées à des sujets sains normaux et/ou des valeurs associées à une sévérité connue du cancer du poumon et/ou des valeurs obtenues chez ledit patient à un moment antérieur et/ou une valeur seuil prédéterminée.

2. Procédé selon la revendication 1, dans lequel la valeur seuil prédéterminée est d'au moins 9,0 ng/mL.

3. Procédé selon les revendications 1 ou 2, dans lequel l'immunodosage est un dosage par compétition ou un dosage en sandwich.

4. Procédé selon les revendications 1 à 3, dans lequel l'immunodosage est un dosage immuno-enzymatique de type ELISA ou un dosage radio-immunologique.

5. Procédé permettant de déterminer si un patient répond positivement à un traitement contre le cancer du poumon, dans lequel ledit procédé comprend la mise en œuvre du procédé selon les revendications 1 à 4 afin de quantifier la quantité de peptides comprenant la séquence d'acides aminés N-terminale LLARDFEKNY (SEQ ID NO: 1) dans au moins deux échantillons biologiques, lesdits échantillons biologiques ayant été obtenus chez ledit patient à un premier moment et à au moins un moment ultérieur pendant une période d'administration du traitement audit patient, et dans lequel une réduction de la quantité de peptides comprenant la séquence d'acides aminés N-terminale LLARDFEKNY (SEQ ID NO: 1) entre ledit premier moment et ledit au moins un moment ultérieur pendant la période de traitement indique une réponse positive dudit patient audit traitement.
